# EUROPÄISCHE PATENTSCHRIFT

(11) **EP 3 849 436 B1**
(45) Veröffentlichungstag und Bekanntmachung des Hinweises auf die Patenterteilung: **26.11.2025**
(21) Anmeldenummer: 19773359.5
(22) Anmeldetag: 09.09.2019
(51) Int. Cl.: A61B 17/16

(54) **CHIRURGISCHES WERKZEUG MIT LAGERUNG**
SURGICAL TOOL WITH BEARING
OUTIL CHIRURGICAL AVEC PALIER

(30) Priorität: 10.09.2018 DE 102018122025
(43) Veröffentlichungstag der Anmeldung: 21.07.2021
(73) Patentinhaber: Aesculap AG, 78532 Tuttlingen (DE)
(72) Erfinder: HÖGERLE, Roland-Alois, 78532 Tuttlingen (DE); PFISTER, Ralf, 78647 Trossingen (DE); BÜRK, André, 78056 Villingen-Schwenningen (DE)
(74) Vertreter: Winter, Brandl - Partnerschaft mbB
(86) Internationale Anmeldenummer: PCT/EP2019/073994
(87) Internationale Veröffentlichungsnummer: WO 2020/053149

(56) Entgegenhaltungen:
- EP-A1- 2 623 049
- WO-A1-2018/075925
- DE-A1- 102008 045 179
- US-B2- 9 175 723
- US-E- R E29 736

## Beschreibung

### Technisches Gebiet

Die vorliegende Offenbarung betrifft ein chirurgisches Werkzeug mit Lagerung.

### Hintergrund der Erfindung

Aus dem Stand der Technik sind chirurgische Werkzeuge, wie beispielsweise chirurgische Fräswerkzeuge oder Bohrwerkzeuge bekannt, die einen Werkzeugschaft mit einem proximalen und einem distalen Endabschnitt aufweisen, welches ein Arbeitsende mit einem Effektor ist. Das Werkzeug ist mit seinem proximalen Endabschnitt über eine Kopplungsstruktur mit einem Antrieb in einem Gehäuse dreh-und axialfest koppelbar, um eine Rotationsbewegung des Antriebs auf das Werkzeug zu übertragen, das sich dann im Fall eines aktiv bedienten Gerätes um seine Längsachse dreht. Zur erforderlichen Lagerung des Werkzeugs gegenüber dem Gehäuse weist das Gehäuse ein darin fest integriertes Drehlager auf. Die Werkzeuge sind üblicherweise austauschbar mit dem Gehäuse verbunden, sodass das Gehäuse mehrfach verwendet werden kann und mit verschiedenen bzw. verschiedenartigen Werkzeugen bestückt werden kann.

### Stand der Technik

Ein derartiges Werkzeug ist beispielsweise aus US RE 29 736 bekannt, wobei das Werkzeug als chirurgischer Bohrer ausgeführt ist. Der Werkzeugschaft weist nahe seinem proximalen Ende eine umlaufende Laufrille auf, in der in einem mehrteiligen Gehäuse fest aufgenommene Kugeln zur Lagerung des Werkzeugschaftes gegenüber dem Gehäuse um laufen können. Der Werkzeugschaft ist gegenüber dem Gehäuse axial verschieblich. In dem Fall, in dem die Kugeln defekt sind, muss der Teil des Gehäuses, in dem die Kugeln aufgenommen sind, ausgetauscht werden.

Des Weiteren ist aus US 9 175 723 B2 ein chirurgisches Werkzeug mit Lagerung bekannt. Hier ist das Werkzeug zu seinem proximalen Endabschnitt hin durch mehrere Kugellager drehbar in einem Gehäuseschaft gelagert. Zu seinem distalen Endabschnitt hin ist das Werkzeug über Nadellager in dem Gehäuseschaft gelagert. Alle Drehlager sind fest im Gehäuseschaft bzw. im Gehäuse integriert.

Darüber hinaus zeigt WO 2018 / 075 925 A1 ein System für intramedulläre Präparationen mit einem Werkzeug mit einer flexiblen Antriebswelle, die ein proximales und ein distales Ende aufweist, sowie einem Schneidkopf, der betriebsmäßig mit dem distalen Ende der flexiblen Antriebswelle verbunden ist.

Dabei entsteht das Problem, dass derartige Drehlager, die Werkzeuge im Gehäuse lagern und Verschleißteile sind, im Gehäuse großen Belastungen ausgesetzt sind, wie beispielsweise Korrosion, Verschmutzung und Überbelastung. Gleichzeitig werden die Gehäuse, in denen die Drehlager integriert sind, vielfach verwendet. Dadurch kann es zu Schäden am Drehlager kommen, sodass die Drehlager in einem defekten Zustand sind und repariert oder ausgetauscht werden müssen. Für die Zeitspanne der Reparatur bzw. des Austauschs kann das Gehäuse bzw. Handstück nicht seiner bestimmungsgemäßen Verwendung zugeführt werden, sodass einem Anwender des Werkzeugs Zeitaufwand und Kosten entstehen.

Es ist daher ein Ziel der Erfindung, die Verfügbarkeit eines Gehäuses mit einem Antrieb, der mit einem chirurgischen Werkzeug koppelbar ist, in Bezug auf das zumindest eine Lager, welches das Werkzeug in dem Gehäuse lagert, zu erhöhen.

### Zusammenfassung der Erfindung

Zur Lösung der gestellten Aufgabe ist ein chirurgisches Werkzeug gemäß Anspruch 1 vorgesehen. D.h. es ist ein chirurgisches Werkzeug, insbesondere ein chirurgisches Fräs- oder Bohrwerkzeug, mit einem Werkzeugschaft, der an seinem distalen Endabschnitt einen Effektor und an seinem proximalen Endabschnitt eine Kopplungsstruktur aufweist, vorgesehen. Die Kopplungsstruktur ist dabei dafür vorgesehen, mit einem Antrieb wahlweise dreh- und axialfest gekoppelt zu werden, um so eine Rotation des Antriebs auf den Werkzeugschaft zu übertragen. Darüber hinaus weist das chirurgische Werkzeug wenigstens ein Drehlager auf, welches zur drehbaren Abstützung des Werkzeugschafts an einem Gehäuse, vorzugsweise Handstück, vorgesehen ist, wobei das Drehlager mit dem Werkzeugschaft zu einer Einheit verbunden ist, die in ihrer Gesamtheit in das Gehäuse für ein Kuppeln mit dem Antrieb einführbar und für ein Entkuppeln von dem Antrieb aus dem Gehäuse herausziehbar ist.

Mit anderen Worten ausgedrückt, ist ein chirurgisches Werkzeug vorgesehen, insbesondere chirurgisches Fräs- oder Bohrwerkzeug, mit einem Werkzeugschaft, der an seinem distalen Endabschnitt einen Effektor und an seinem proximalen Endabschnitt eine Kopplungsstruktur aufweist, die dafür vorgesehen und angepasst ist, mit einem in einem Gehäuse (oder Gehäuseschaft), vorzugsweise Handstück, aufgenommenen Antrieb wahlweise dreh- und axialfest mit diesem gekoppelt zu werden, um so eine Rotation des Antriebs auf den Werkzeugschaft zu übertragen, wofür zwischen dem Werkzeugschaft und dem Gehäuse, vorzugsweise Handstück, wenigstens ein Drehlager zur drehbaren Abstützung des Werkzeugschafts am Gehäuse, vorzugsweise Handstück, angeordnet ist. Erfindungsgemäß ist das Drehlager mit dem Werkzeugschaft zu einer Einheit verbunden, die in ihrer Gesamtheit in das Gehäuse für ein Kuppeln mit dem Antrieb einführbar und für ein Entkuppeln von dem Antrieb aus dem Gehäuse herausziehbar ist.

In noch anderen Worten ist ein chirurgisches Werkzeug vorgesehen, insbesondere chirurgisches Fräswerkzeug, mit einem Werkzeugschaft sowie einem distalen und einem proximalen Endabschnitt. Das Werkzeug ist mit seinem proximalen Endabschnitt auswechselbar in ein Gehäuse / Handstück eingeführt und mit einem Antrieb im Gehäuse dreh- und axialfest gekoppelt, um um seine Längsachse die Rotationsbewegung, die der Antrieb erzeugt, ausführen zu können. Das Werkzeug ist über zumindest ein Lager/Drehlager/Lagerelement in dem Gehäuse gelagert. Das Drehlager ist dabei lösbar, vorzugsweise als Rastverbindung oder Steckverbindung, mit dem Gehäuse und (axial-)fest mit dem Werkzeug verbunden, derart, dass das Drehlager mit dem Werkzeugschaft eine Einheit bildet.

Somit können die Drehlager unabhängig vom Handstück bzw. Gehäuse mit dem Werkzeug zusammen erneuert werden und müssen nicht ein Serviceintervall des Gehäuses überstehen. Somit kann der Ausfall von Drehlagern deutlich reduziert werden. In dem Fall, dass ein Drehlager defekt ist, wird das Werkzeug mit fest integriertem Drehlager das in diesem Fall unlösbar an/auf dem Werkzeugschaft aufgenommen ist, ausgetauscht und somit kann das entsprechende Handstück weiterhin seiner zweckmäßigen Verwendung zugeführt werden. Der Austausch des Werkzeugs mit Drehlager für das Handstück kann ein Anwender bei Bedarf mit jedem Werkzeugwechsel einfach und schnell selbständig durchführen. Dadurch wird eine hohe Verfügbarkeit und Zuverlässigkeit der Handstücke gewährleistet. Durch das unabhängig vom Handstück austauschbare Drehlager entsteht eine Kosten- und/oder Zeitersparnis für den Anwender des erfindungsgemäßen chirurgischen Werkzeugs.

Bevorzugt werden als Drehlager Wälzlager verwendet, die einen Lagerinnenabschnitt mit Führung, einen Außenabschnitt mit Führung und dazwischen einen Käfig mit darin gelagerten Wälzkörpern aufweisen, die zwischen den Führungen des Lagerinnenabschnittes und des Lageraußenabschnittes angeordnet sind. Die Wälzlager, insbesondere Kugellager, sind meist ringförmig ausgeführt und so am Werkzeugschaft angebracht, dass das Drehlager und der Werkzeugschaft koaxial zueinander sind. Alternativ können als Drehlager auch Gleitlager verwendet werden, die keine Wälzkörper zwischen ihrem Lagerinnen- und Lageraußenabschnitt aufweisen.

Erfindungsgemäß ist das Drehlager mit dem Werkzeugschaft integral ausgebildet, insbesondere auf den Werkzeugschaft aufgepresst, sodass Werkzeugschaft bzw. Werkzeug und Drehlager eine unlösbare Einheit ausbilden und das Werkzeug vorzugsweise ein Einmalwerkzeug ist.

Wenn das Drehlager integral mit dem Werkzeugschaft ausgebildet ist, kann das Drehlager vorteilhafterweise mit jedem Werkzeugwechsel erneuert werden, sodass das Werkzeug ein Einmalwerkzeug ist. Eine Art, das Drehlager mit dem Werkzeugschaft unlösbar zu verbinden, ist ein Aufpressen des Lagerinnenabschnittes auf den Werkzeugschaft. In dem Fall, in dem Drehlager und Werkzeug eine unlösbare Einheit ausbilden, kann das Drehlager ein besseres Laufverhalten erzielen und es entsteht kein Schlupf zwischen Drehlager und Werkzeugschaft. Weiterhin erhöht sich die Tragfähigkeit und damit auch die Belastbarkeit des Drehlagers.

Nicht Teil dieser Erfindung ist eine zu einem mit dem Werkzeug integral ausgebildeten Drehlager alternative Ausführung, bei welcher das Drehlager separat von dem Werkzeugschaft ausgebildet sein kann, insbesondere auf den Werkzeugschaft aufgeschoben sein, sodass Werkzeugschaft bzw. Werkzeug und Drehlager eine lösbare Einheit ausbilden.

Der Vorteil in dieser Ausführungsform besteht darin, dass das Werkzeug unabhängig von einem Verschleiß des Drehlagers ist und mehrfach verwendet werden kann. Außerdem kann ein Anwender das Drehlager bei Bedarf einfach selbst, beispielsweise unter Verwendung eines Kugellagermagazins, austauschen.

In einer weiteren Ausführungsform kann der Werkzeugschaft zwischen dem proximalen und dem distalen Endabschnitt zumindest einen radial umlaufenden, und insbesondere koaxial und/oder damit integral ausgebildeten, Vorsprung zur axialen Sicherung des Drehlagers aufweisen. Dabei kontaktiert der Vorsprung mit seiner dem proximalen Endabschnitt zugewandten Seite direkt das Drehlager.

Der Vorsprung dient als Schutz für das Drehlager und verhindert eine unerwünschte Axialverschiebung des Drehlagers in Richtung des distalen Endabschnittes. Ein solcher Vorsprung ist vor allem in dem Fall erforderlich, in dem das Drehlager separat von dem Werkzeugschaft ausgebildet, beispielsweise aufgeschoben, ist. Weiterhin verhindert der Vorsprung in dem Fall, dass ein Wälzlager als Drehlager verwendet wird, den Verlust der Wälzkörper.

Vorzugsweise kann der Vorsprung in Axialrichtung konisch ausgebildet sein und dabei vergrößert sich die Radialabmessung des Vorsprungs vom distalen Endabschnitt in Richtung des proximalen Endabschnittes hin, vorzugsweise bis die Radialabmessung des Vorsprungs zumindest so groß wie die Außen-Radialabmessung des Drehlagers ist. Alternativ dazu kann der Vorsprung plan ausgebildet sein. Der Vorsprung kann weiterhin zumindest nur so groß sein, dass seine Radialabmessung der Radialabmessung eines Lagerinnenabschnittes entspricht. Je größer der Vorsprung ist, desto effektiver sichert er das Drehlager.

Weiter bevorzugt weist der Werkzeugschaft, der unlösbar mit dem Drehlager verbunden ist, zumindest eine radial umlaufende Laufrille auf, die das Drehlager aufnimmt, sodass die Laufrille ein Teil des Drehlagers ist.

Die Ausbildung einer Laufrille am Werkzeugschaft als Teil des Drehlagers ist eine Alternativlösung zum Aufpressen des Lagerinnenabschnittes auf den Werkzeugschaft. Im Falle einer derartigen Laufrille entfällt der Lagerinnenabschnitt des Drehlagers und das Drehlager ist nicht wechselbar am Werkzeug montiert, sondern kann erst bei einem Werkzeugwechsel mit dem Werkzeug ersetzt werden. Im Falle eines Wälzlagers werden die Wälzkörper mit dem Lageraußenabschnitt direkt auf den Werkzeugschaft aufgebracht. Durch den nicht vorhandenen Lagerinnenabschnitt entsteht der Vorteil, dass das Drehlager eine Komponente weniger aufweist, die folglich nicht verschleißen oder brechen kann, was somit eine längere Verwendung und Zuverlässigkeit des Drehlagers hervorrufen kann. Auch die Produktionskosten für ein derartiges Drehlager sind im Vergleich zu herkömmlichen Drehlagern mit Lagerinnenabschnitt geringer. In dieser bauraumoptimierten Ausführung ist das Drehlager ein fester Bestandteil des Werkzeugs. In dem Fall, dass die Laufrille die Funktion des Lagerinnenabschnitts übernimmt, kann außerdem die Baugröße des Drehlagers im Vergleich zu einem herkömmlichen Drehlager reduziert werden und führt somit zu schlankeren Arbeitsenden. Alternativ dazu kann die Tragzahl des Drehlagers erhöht werden, was zu einer erhöhten Lagerlebensdauer und -stabilität beiträgt. Eine höhere Tragzahl erlaubt außerdem einen größeren Abstand zwischen Drehlager und distalem Endabschnitt. Somit sind sowohl die Baugrößenreduzierung als auch die Tragzahlerhöhung des Drehlagers vorteilhaft für den Zugang und die Sicht bei Verwendung des chirurgischen Werkzeugs.

Weiterhin kann die Laufrille in Form einer nutähnlichen Vertiefung in der Außenumfangsfläche des Werkzeugschaftes oder als ein Bereich zwischen zwei in Axialrichtung voneinander beabstandeten, radial umlaufenden Lippen ausgebildet sein, die sich von der Außenumfangsfläche des Werkzeugschafts aus, beispielsweise senkrecht, erheben.

Eine solche Nut kann einfach und kostengünstig in den Werkzeugschaft eingebracht werden (bspw. durch Drehen). An der Stelle, an der die Laufrille als Nut ausgebildet ist, ist die Stärke des Werkzeugschafts geringer als im daran angrenzenden Bereich. Damit kann es sein, dass die Nut eine Sollbruchstelle für den Werkzeugschaft darstellt, zumindest aber die Lastbeständigkeit des Werkzeugs minimiert. Alternativ kann die Laufrille auch so ausgeführt sein, dass sich von der Oberfläche des Werkzeugschaftes aus zwei radial umlaufende Lippen erheben, die in der Axialrichtung des Werkzeugschaftes voneinander beabstandet sind. Die Laufrille ist damit in dem Bereich ausgebildet, den die beiden Lippen einschließen. In diesem Fall ist der Werkzeugschaft radial unterhalb der Laufrille und im axial angrenzenden Bereich von einer gleichbleibenden Materialstärke. In jedem Fall muss die Laufrille so ausgebildet sein, dass sie die Aufgabe der Führung eines Lagerinnenabschnittes erfüllt.

In einer bevorzugten Ausführungsform weist das Drehlager an seiner Außenumfangsfläche zumindest einen ersten Rastabschnitte auf, das Gehäuse sieht mit dem zumindest einen ersten Rastabschnitt zusammenwirkenden zweiten Rastabschnitt vor und der erste und zweite Rastabschnitt greifen in einem Rastzustand ineinander und tragen somit zur Lagerung des Werkzeugschaftes gegenüber dem Gehäuse bei.

Dabei kann der erste Rastabschnitt eine Rastnase sein und der zweite Rastabschnitt kann eine Vertiefung sein oder umgekehrt. Es ist die Aufgabe dieser Rastverbindung, zu verhindern, dass sich die Rotationsbewegung des Antriebs bzw. des Werkzeugs auf den Lageraußenabschnitt überträgt. Der Lageraußenabschnitt kann also keine Rotationsbewegung ausführen. Wird das Werkzeug mit dem fest angebrachten Drehlager in das Gehäuse eingeführt, rastet die Rastnase in einem Rastzustand in die dafür vorgesehen Vertiefung ein. Zum Lösen der Rastverbindung zwischen Gehäuse und Lager, muss nur eine Zugkraft auf das Werkzeug ausgeübt werden, durch die die Rastverbindung gelöst werden kann. Dies trägt zu einem unkomplizierten und schnellen Werkzeugeinbau bzw. einer unkomplizierten und schnellen Werkzeugentfernung bei.

Weiterhin kann das Drehlager ein ein-, zwei- oder mehrreihig ausgeführtes Drehlager sein. Darüber hinaus kann das Drehlager auch ein ein,- zwei- oder mehrreihiges Gleitlager sein, das im Gegensatz zu Wälzlagern keine Wälzkörper aufweist.

Mehrreihig ausgeführte Drehlager weisen eine höhere Tragfähigkeit auf und haben damit eine erhöhte Lebensdauer und Belastbarkeit als ein einreihiges Drehlager. Gleitlager sind vor allem für Lowspeed- (geringe Werkzeugumdrehungszahlen) Anwendungen geeignet.

Erfindungsgemäß ist weiterhin ein chirurgisches Werkzeugsystem mit einem Gehäuse und einem chirurgischen Werkzeug vorgesehen, welches mit einem Antrieb im Gehäuse dreh-und axialfest koppelbar ist, um eine vom Antrieb erzeugte Rotationsbewegung zu übernehmen, und an seinem Werkzeugschaft ein damit fest verbundenes Drehlager aufweist, das das Werkzeug gegenüber dem Gehäuse lagert.

### Kurzbeschreibung der Figuren

Im Folgenden sind Ausführungsformen des erfindungsgemäßen chirurgischen Werkzeugs unter Bezug auf die beigefügten Zeichnungen im Detail beschrieben. Dabei werden gleichen Elementen dieselben Bezugszeichen zugewiesen. Die Ausführungsformen sind nur beispielhaft und die Erfindung ist nicht darauf begrenzt.
Fig. 1A zeigt ein chirurgisches Werkzeug mit einem am Werkzeug angebrachten Drehlager;
Fig. 1B ist ein Ausschnitt aus Fig. 1A und zeigt ein Drehlager sowie einen Teil eines Werkzeugschaftes, der das Drehlager trägt;
Fig. 2A zeigt einen Teil eines Werkzeugschaftes mit darauf angebrachtem Drehlager und mit einem an das Drehlager angrenzenden Vorsprung;
Fig. 2B zeigt einen Teil eines Werkzeugschaftes mit darauf angebrachtem Drehlager und mit einem an das Drehlager angrenzenden, modifizierten Vorsprung;
Fig. 2C zeigt einen Teil eines Werkzeugschaftes mit darauf angebrachtem Drehlager und mit einem an das Drehlager angrenzenden, weiter modifizierten Vorsprung;
Fig. 2D zeigt einen Teil eines Werkzeugschaftes mit darauf angebrachtem Drehlager und mit einem an das Drehlager angrenzenden, weiter modifizierten Vorsprung;
Fig. 3A zeigt einen Teil eines Werkzeugschaftes mit einem Drehlager und einer als Nut ausgebildeten Laufrille;
Fig. 3B zeigt einen Teil eines Werkzeugschaftes mit einem Drehlager und einer zwischen zwei Lippen ausgebildeten Laufrille;
Fig. 4 zeigt einen Teil eines Werkzeugschaftes mit einem Doppellager

### Beschreibung der Ausführungsbeispiele

Nachstehend werden Ausführungsbeispiele der vorliegenden Offenbarung auf der Basis der zugehörigen Figuren beschrieben.

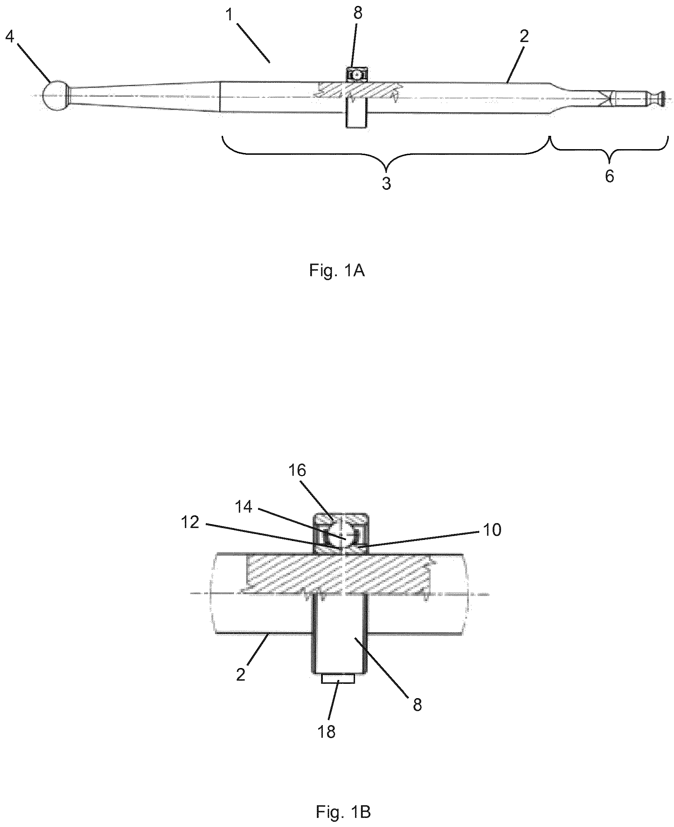

Fig. 1A zeigt ein chirurgisches Fräswerkzeug 1 mit einem Werkzeugschaft 2, der einen Hauptabschnitt 3 aufweist, mit einem Effektor 4 am distalen Endabschnitt und einer Kopplungsstruktur 6 am proximalen Endabschnitt. Der Effektor 4 ist hier als Fräser ausgeführt, kann aber auch ein Bohrer sein. Der Werkzeugschaft 2 läuft zu seinem distalen Endabschnitt hin konisch zu. Die Kopplungsstruktur 6 ist in ihrer Radialabmessung schmaler als der Werkzeugschaft 2, um einfach in ein Gehäuse bzw. Handstück (nicht gezeigt) eingeführt werden zu können. Im Gehäuse befindet sich ein Antrieb (nicht gezeigt), mit dem das Werkzeug 1 über die Kopplungsstruktur 6 so gekoppelt werden kann, dass es eine vom Antrieb erzeugte Rotationsbewegung übernimmt. Im Falle einer Rotation des Werkzeugs 1 ist eine Lagerung zwischen Werkzeug 1 und Gehäuse erforderlich. Erfindungsgemäß befindet sich daher ein Drehlager 8 an dem Werkzeugschaft 2 und ist fest mit dem Werkzeugschaft 2 verbunden. Das Drehlager 8 ist hier auf oder nahe der Mittelquerachse des Werkzeugschafts 2, auf jeden Fall jedoch in dessen Hauptabschnitt 3, angeordnet.

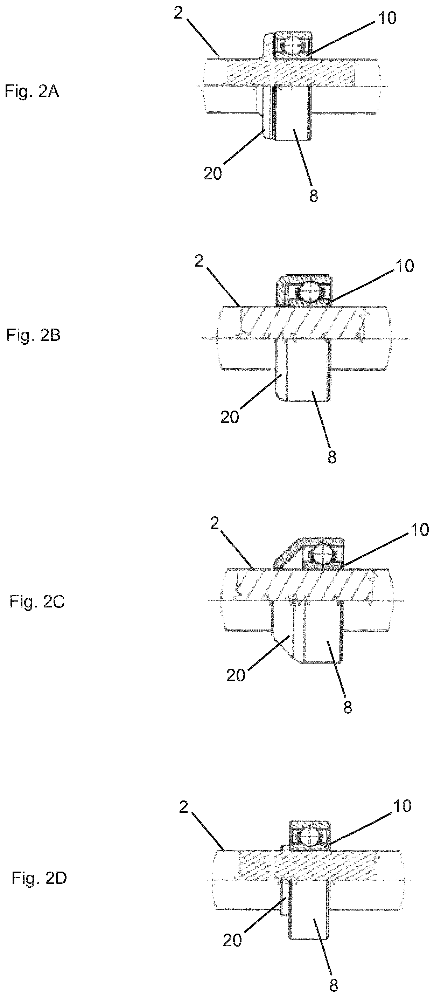

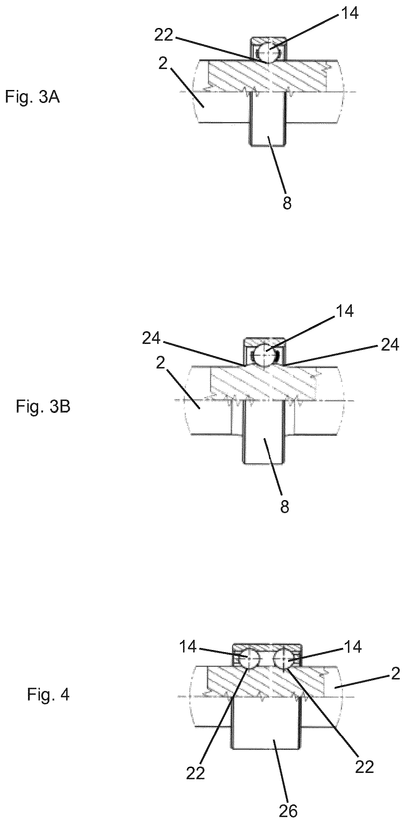

Fig. 1B zeigt einen Ausschnitt aus Fig. 1A. Das mit dem Werkzeugschaft 2 zu einer Einheit verbundene Drehlager 8 ist in dieser Ausführungsform als Kugellager ausgeführt, kann aber alternativ auch ein anderes Wälzlager oder aber ein Gleitlager sein. Das Drehlager weist einen Lagerinnenabschnitt 10, eine Führung 12 am Lagerinnenabschnitt, Kugeln (Wälzkörper) 14 und einen Außenabschnitt 16 auf. In allen Figuren (Fig. 1A, 1B und Fig. 2A - 2D), in denen das auf den Werkzeugschaft 2 montierte Kugellager 8 mit Lagerinnenabschnitt 10 dargestellt ist, kann das Drehlager 8 auf den Werkzeugschaft 2 entweder aufgeschoben (lösbare Verbindung) oder aufgepresst (unlösbare Verbindung) sein. In den anderen Fällen (Fig. 3A, 3B und Fig. 4), in denen das Kugellager 8 ohne Lagerinnenabschnitt 10 über eine Laufrille am Werkzeugschaft 2 montiert ist, ist diese Laufrille ein Teil des Kugellagers 10 und ersetzt dessen Lagerinnenabschnitt 10.

Das Kugellager 8, wie es in Fig. 1B gezeigt ist, weist an seiner Außenumfangsfläche eine axial verlaufende Rastnase 18 auf. Allerdings ist denkbar, dass das Kugellager mehrere Rastnasen 18 aufweist. Die Rastnasen 18 sind dazu vorgesehen, in entsprechend dafür vorgesehene Vertiefungen (nicht gezeigt) im Gehäuse einzugreifen, um zu verhindern, dass der Außenabschnitt 16 des Kugellagers 8 die Rotationsbewegung des Antriebs übernimmt, um zur Lagerung zwischen Werkzeug und Gehäuse beizutragen. Der Lagerinnenabschnitt 10 ist fest mit dem Werkzeugschaft 2 verbunden: entweder ist der Lagerinnenabschnitt 10 auf den Werkzeugschaft 2 aufgeschoben und damit das Drehlager 8 vom Werkzeug 1 lösbar/separat ausgebildet oder der Lagerinnenabschnitt 10 ist fest auf den Werkzeugschaft 2 aufgepresst, sodass das Werkzeug 1 und das Drehlager 8 unlösbar/integral ausgebildet sind. In jedem Fall bilden das Werkzeug 1 bzw. der Werkzeugschaft 2 und das Kugellager 8 eine Einheit aus.

Fig. 2A zeigt einen Ausschnitt des Werkzeugschaftes 2, der das Kugellager 8 aufweist. In Richtung zum distalen Endabschnitt des Werkzeugs 1 hin grenzt direkt an das Kugellager 8 ein Vorsprung 20 an. Der Vorsprung 20 ist so ausgebildet, dass seine maximale Radialabmessung an der Seite, die mit dem Kugellager 8 in Kontakt steht, so groß wie die Außen-Radialabmessung des Kugellagers 8 ist. In dieser Ausführungsform ist der Vorsprung 20 flanschartig ausgebildet. Bei der zweckgemäßen Anwendung des Fräswerkzeugs 1 dient der Vorsprung 20 als Sicherung gegen eine unerwünschte Verschiebung des Drehlagers zum Effektor 4 hin. Der Vorsprung 20 kann auf den Werkzeugschaft aufgeschobene (damit separat ausgebildete) und aufgepresste (damit integral ausgebildete) Drehlager vor dem Verrutschen zum Effektor hin schützen. Außerdem verhindert der Vorsprung 20 einen Verlust der Kugeln 14. Somit hat der Vorsprung 20 eine Schutzfunktion für das Kugellager 8. Der Vorsprung 20 ist integral mit dem Werkzeugschaft 2 ausgebildet und kontaktiert mit einer (dem proximalen Endabschnitt zugewandten) Stirnseite das Drehlager 8.

Fig. 2B zeigt den Vorsprung 20 in einer alternativen Ausführungsform. Hier ist der Vorsprung 20 plan ausgebildet und seine Radialabmessung ist über seine gesamte Höhe gleich und entspricht der Radialabmessung des Außenumfangs des Kugellagers 8.

Fig. 2C zeigt eine weitere alternative Ausführung des Vorsprungs 20. Hier ist der Vorsprung 20 konisch ausgebildet, und zwar derart, dass der Vorsprung 20 sich in seiner Radialabmessung ausgehend von der Radialabmessung an der Seite, die mit dem Kugellager 8 in Kontakt steht, die hier so groß wie die Außen-Radialabmessung des Kugellagers 8 ist, zum distalen Endabschnitt hin auf eine Radialabmessung kontinuierlich verjüngt, die hier nur geringfügig größer als die Radialabmessung des Werkzeugschaftes 2 ist.

Fig. 2D zeigt einen gegenüber den bisher beschriebenen Ausführungsformen des Vorsprungs 20 modifizierten Vorsprung 20. Dessen Radialabmessung ist nur so groß wie die Außenradialabmessung des Lagerinnenabschnittes 10, und sichert somit nicht das komplette Drehlager 8, sondern nur den Lagerinnenabschnitt 10 vor einer Verschiebung in Axialrichtung zum distalen Endabschnitt hin. Ein derartig gegenüber den anderen Ausführungsformen verkürzter Vorsprung kann flanschartig, plan oder konisch ausgeführt sein.

Fig. 3A zeigt einen Teil des Werkzeugschaftes 2 mit einer Laufrille in Form einer radial umlaufenden Nut 22, die als Führung 12 für die Kugeln 14 des Kugellagers 8 dient. Der Lagerinnenabschnitt 10 des Kugellagers 8 ist daher unnötig und entfällt. Die Nut 22 ist gegenüber der äußeren Oberfläche des Werkzeugschaftes 2 als eine Vertiefung ausgebildet, sodass der Werkzeugschaft 2 an dieser Stelle schmaler als im restlichen Hauptabschnitt 3 des Werkzeugschaftes 2 ist.

Fig. 3B zeigt einen Teil des Werkzeugschaftes 2 mit einer gegenüber Fig. 3A alternativen Ausführungsform der Laufrille, die als der Bereich zwischen zwei radial umlaufenden und in Axialrichtung des Werkzeugschaftes 2 voneinander beabstandeten Lippen 24 definiert ist. Die hügelförmig ausgebildeten Lippen 24 erheben sich von der äußeren Oberfläche des Werkzeugschaftes 2 aus und sind so ausgebildet und voneinander beabstandet, dass sie die Kugeln 14 des Drehlagers 8 aufnehmen können. Damit hat der Werkzeugschaft 2 an der Stelle der Laufrille dieselbe Stärke wie im restlichen Werkzeugschafthauptabschnitt 3 und ist auf Höhe der zwei Lippen 24 sogar verstärkt.

Auch wenn dies nicht dargestellt ist, kann der Werkzeugschaft 2 mehrere integral oder separat ausgebildete Drehlager 8 aufweisen und somit auch mehrere gleich- oder verschiedenartig ausgebildete Vorsprünge 20 oder Laufrillen aufweisen.

Fig. 4 zeigt einen Teil des Werkzeugschaftes 2, der mit einem Doppel-Kugellager 26 eine Einheit ausbildet. Das Doppel-Kugellager 26 ist ein zweireihiges Kugellager. Der Werkzeugschaft 2 weist zur Führung der Kugeln 14 zwei radial umlaufende und in Axialrichtung voneinander beabstandete Laufrillen in Form von zwei Nuten 22 auf, in denen jeweils ein Satz an Kugeln 8 umläuft. Das Doppel-Kugellager 26 weist daher keinen Lagerinnenabschnitt 10 auf. Auch wenn es nicht dargestellt ist, kann ein drei- oder mehrreihiges Drehlager verwendet werden. Auch ist denkbar, dass die zwei- bzw. mehrreihigen Drehlager einen Lagerinnenabschnitt aufweisen und auf den Werkzeugschaft 2 aufgeschoben oder aufgepresst sind, und durch einen Vorsprung 20 geschützt werden.

Es ist allgemein möglich, die hier aufgezeigten Ausführungsformen untereinander zu kombinieren, solange dies technisch sinnvoll ist.

### Liste der Bezugszeichen:

- 1:: Chirurgisches Werkzeug
- 2:: Werkzeugschaft
- 3:: Werkzeugschafthauptabschnitt
- 4:: Effektor am distalen Endabschnitt des Werkzeugs
- 6:: Kopplungsstruktur am proximalen Endabschnitt des Werkzeugs
- 8:: Drehlager
- 10:: Lagerinnenabschnitt
- 12:: Führung für Wälzkörper am Lagerinnenabschnitt
- 14:: Wälzkörper (Kugeln)
- 16:: Lageraußenabschnitt
- 18:: Rastnase
- 20:: Vorsprung
- 22:: Nut
- 24:: Lippe
- 26:: mehrreihiges Lager

## Patentansprüche

1. Chirurgisches Werkzeug (1), insbesondere chirurgisches Fräs- oder Bohrwerkzeug, mit einem Werkzeugschaft (2), der an seinem distalen Endabschnitt einen Effektor (4) und an seinem proximalen Endabschnitt eine Kopplungsstruktur (6) aufweist, die dafür vorgesehen ist, mit einem Antrieb wahlweise dreh- und axialfest gekoppelt zu werden, um so eine Rotation des Antriebs auf den Werkzeugschaft (2) zu übertragen,
und wenigstens einem Drehlager (8), das zur drehbaren Abstützung des Werkzeugschafts (2) an einem Gehäuse, vorzugsweise Handstück, vorgesehen ist, wobei das Drehlager (8) mit dem Werkzeugschaft (2) zu einer Einheit verbunden ist, die in ihrer Gesamtheit in das Gehäuse für ein Kuppeln mit dem Antrieb einführbar und für ein Entkuppeln von dem Antrieb aus dem Gehäuse herausziehbar ist,
**dadurch gekennzeichnet, dass** das Drehlager (8) mit dem Werkzeugschaft (2) integral ausgebildet ist, insbesondere auf den Werkzeugschaft (2) aufgepresst ist, sodass der Werkzeugschaft (2) des Werkzeugs (1), vorzugsweise ein Einmalwerkzeug, und das Drehlager (8) zu einer unlösbaren Einheit verbunden sind.

2. Chirurgisches Werkzeug (1) gemäß Anspruch 1, **dadurch gekennzeichnet, dass** der Werkzeugschaft (2) zwischen dem proximalen und dem distalen Endabschnitt zumindest einen radial umlaufenden, und insbesondere koaxial und/oder damit integral ausgebildeten, Vorsprung (20) zur axialen Sicherung des Drehlagers (8) aufweist, derart, dass der Vorsprung (20) mit seiner dem proximalen Endabschnitt zugewandten Seite direkt das Drehlager (8) kontaktiert.

3. Chirurgisches Werkzeug (1) gemäß Anspruch 2, **dadurch gekennzeichnet, dass** der Vorsprung (20) in Axialrichtung konisch ausgebildet ist und sich dabei die Radialabmessung des Vorsprungs (20) vom distalen Endabschnitt in Richtung des proximalen Endabschnittes hin vergrößert.

4. Chirurgisches Werkzeug (1) gemäß Anspruch 1, **dadurch gekennzeichnet, dass** der Werkzeugschaft (2) zumindest eine radial umlaufende Laufrille aufweist, die ein Teil des Drehlagers (8) ist.

5. Chirurgisches Werkzeug (1) gemäß Anspruch 4, **dadurch gekennzeichnet, dass** die Laufrille in Form einer Nut (22) oder als ein Bereich zwischen zwei in Axialrichtung voneinander beabstandeten, radial umlaufenden Lippen (24) ausgebildet ist, die sich von der Außenumfangsfläche des Werkzeugschafts (2) aus erheben.

6. Chirurgisches Werkzeug (1) gemäß einem der vorangegangenen Ansprüche 1 bis 5, **dadurch gekennzeichnet, dass** das Drehlager (8) an seiner Außenumfangsfläche zumindest einen ersten Rastabschnitt aufweist,
das Gehäuse mit dem zumindest einen ersten Rastabschnitt zusammenwirkenden zweiten Rastabschnitt vorsieht, und
der erste und der zweite Rastabschnitt in einem Rastzustand ineinandergreifen können und somit zur Lagerung des Werkzeugschaftes (2) gegenüber dem Gehäuse beitragen.

7. Chirurgisches Werkzeug (1) gemäß einem der vorangegangenen Ansprüche 1 bis 6, **dadurch gekennzeichnet, dass** das Drehlager (8) ein zwei- oder mehrreihig ausgeführtes Drehlager (8) ist.

8. Chirurgisches Werkzeugsystem mit einem Gehäuse, einem Antrieb im Gehäuse, und dem chirurgischen Werkzeug (1), gemäß einem der vorangegangenen Ansprüche 1 bis 7, **dadurch gekennzeichnet, dass** das Werkzeug (1) mit dem Antrieb im Gehäuse dreh-und axialfest koppelbar ist, um eine vom Antrieb erzeugte Rotationsbewegung zu übernehmen, und an seinem Werkzeugschaft (2) das damit fest verbundenes Drehlager (8) aufweist, das das Werkzeug (1) gegenüber dem Gehäuse lagert.

## Claims

1. A surgical tool (1), in particular a surgical milling or drilling tool, including a tool shaft (2), which has an effector (4) at its distal end portion, and a coupling structure (6) at its proximal end portion, said coupling structure being adapted to be selectively coupled to a drive in a rotationally and axially fixed manner in order to thus transmit rotation of the drive to the tool shaft (2),
and at least one pivot bearing (8) provided for rotatably supporting the tool shaft (2) on a housing, preferably a handpiece, the pivot bearing (8) being connected to the tool shaft (2) to form a unit that can be inserted into the housing in its entirety for coupling to the drive, and that can be withdrawn from the housing for decoupling from the drive,
**characterized in that** the pivot bearing (8) is integrally formed with the tool shaft (2), in particular is pressed onto the tool shaft (2), so that the tool shaft (2) of the tool (1), preferably a disposable tool, and the pivot bearing (8) are connected to form an inseparable unit.

2. The surgical tool (1) according to claim 1, **characterized in that** the tool shaft (2), between the proximal and the distal end portion, has at least one protrusion (20) radially circumferential and, in particular coaxial and/or integrally formed therewith for axially securing the pivot bearing (8) such that the protrusion (20) directly contacts the pivot bearing (8) with its side facing the proximal end portion.

3. The surgical tool (1) according to claim 1, **characterized in that** the protrusion (20) is conical in the axial direction, with the radial dimension of the protrusion (20) increasing from the distal end portion toward the proximal end portion.

4. The surgical tool (1) according to claim 1, **characterized in that** the tool shaft (2) has at least one radially circumferential running groove that is part of the pivot bearing (8).

5. The surgical tool (1) according to claim 4, **characterized in that** the running groove is formed in the shape of a slot (22) or as an area between two radially circumferential lips (24) spaced apart from each other in the axial direction, which rise from the outer circumferential surface of the tool shaft (2).

6. The surgical tool (1) according to any of preceding claims 1 to 5,
**characterized in that** the pivot bearing (8) has at least one first locking section on its outer circumferential surface,
the housing provides a second locking section interacting with the at least one first locking section, and
the first and second locking sections can engage with each other in a locking state and thus contribute to supporting the tool shaft (2) relative to the housing.

7. The surgical tool (1) according to any of preceding claims 1 to 6, **characterized in that** the pivot bearing (8) is a two-row or multi-row pivot bearing (8).

8. A surgical tool system including a housing, a drive in the housing and the surgical tool (1) according to any of preceding claims 1 to 7, **characterized in that** the tool (1) can be coupled to the drive in the housing in a rotationally and axially fixed manner in order to take over a rotational motion generated by the drive, and has a pivot bearing (8) on its tool shaft (2) firmly connected to it, which pivot bearing supports the tool (1) relative to the housing.

## Revendications

1. Outil chirurgical (1), en particulier outil chirurgical de fraisage ou de perçage, avec une tige d'outil (2) qui présente à sa section d'extrémité distale un effecteur (4) et à sa section d'extrémité proximale une structure de couplage (6) qui est prévue pour être couplée au choix de manière non rotative et axialement fixe à un entraînement pour ainsi transmettre une rotation de l'entraînement à la tige d'outil (2),
et au moins un palier rotatif (8) qui est prévu pour supporter de manière rotative la tige d'outil (2) sur un boîtier, de préférence une pièce à main, dans lequel le palier rotatif (8) est connecté avec la tige d'outil (2) à une unité qui peut dans son ensemble être insérée dans le boîtier pour un couplage avec l'entraînement et peut être extraite du boîtier pour un découplage de l'entraînement,
**caractérisé en ce que** le palier rotatif (8) est conçu d'un seul tenant avec la tige d'outil (2), en particulier est pressé sur la tige d'outil (2), de sorte que la tige d'outil (2) de l'outil (1), de préférence un outil à usage unique, et le palier rotatif (8) sont connectés à une unité non détachable.

2. Outil chirurgical (1) selon la revendication 1, **caractérisé en ce que** la tige d'outil (2) présente entre la section d'extrémité proximale et la section d'extrémité distale au moins une saillie (20) s'étendant radialement, et en particulier conçue de manière coaxiale et/ou solidaire avec celle-ci, pour fixer axialement le palier rotatif (8), de sorte que la saillie (20) entre directement en contact avec le palier rotatif (8) avec son côté tourné vers la section d'extrémité proximale.

3. Outil chirurgical (1) selon la revendication 2, **caractérisé en ce que** la saillie (20) est conçue de manière conique en direction axiale et la dimension radiale de la saillie (20) augmente de la section d'extrémité distale en direction de la section d'extrémité proximale.

4. Outil chirurgical (1) selon la revendication 1, **caractérisé en ce que** la tige d'outil (2) présente au moins une gorge de roulement s'étendant radialement, qui fait partie du palier rotatif (8).

5. Outil chirurgical (1) selon la revendication 4, **caractérisé en ce que** la gorge de roulement est conçue sous forme d'une rainure (22) ou d'une zone entre deux lèvres (24) s'étendant radialement, espacées l'une de l'autre en direction axiale, qui s'élèvent à partir de la surface périphérique extérieure de la tige d'outil (2).

6. Outil chirurgical (1) selon l'une quelconque des revendications précédentes 1 à 5, **caractérisé en ce que** le palier rotatif (8) présente sur sa surface périphérique extérieure au moins une première section d'enclenchement,
le boîtier prévoit une seconde section d'enclenchement en coopération avec l'au moins une première section d'enclenchement, et
les première et seconde sections d'enclenchement peuvent s'engager l'une dans l'autre dans un état d'enclenchement et contribuent ainsi au stockage de la tige d'outil (2) en face du boîtier.

7. Outil chirurgical (1) selon l'une quelconque des revendications précédentes 1 à 6, **caractérisé en ce que** le palier rotatif (8) est un palier rotatif (8) réalisé à deux ou plusieurs rangées.

8. Système d'outil chirurgical avec un boîtier, un entraînement dans le boîtier et l'outil chirurgical (1), selon l'une quelconque des revendications précédentes 1 à 7, **caractérisé en ce que** l'outil (1) peut être couplé de manière non rotative et axialement fixe à l'entraînement dans le boîtier pour reprendre un mouvement de rotation généré par l'entraînement, et présente sur sa tige d'outil (2) le palier rotatif (8) auquel il est connecté de manière fixe et qui supporte l'outil (1) en face du boîtier.
